## Europäisches Patentamt
## European Patent Office
## Office européen des brevets

(19)

(11) Veröffentlichungsnummer: **0 016 425**
**B1**

(12)

# EUROPÄISCHE PATENTSCHRIFT

(45) Veröffentlichungstag der Patentschrift:
02.10.85

(21) Anmeldenummer: 80101311.1

(22) Anmeldetag: 13.03.80

(51) Int. Cl.⁴: **G 01 N 33/48,** C 12 Q 1/06,
C 12 M 3/04, C 12 N 5/02

(54) Diagnoseverfahren.

(30) Priorität: 15.03.79 CH 2454/79

(43) Veröffentlichungstag der Anmeldung:
01.10.80 Patentblatt 80/20

(45) Bekanntmachung des Hinweises auf die Patenterteilung:
02.10.85 Patentblatt 85/40

(84) Benannte Vertragsstaaten:
AT BE CH DE FR GB IT LU NL SE

(56) Entgegenhaltungen:
FR - A - 2 370 094

Proc. Nat. Acad. Sci. USA, Vol. 70, No. 2, Febr. 1973, Seiten 369-372
Laboratory Investigation, Vol. 36, No. 1, Seiten 18-25, 1977
Nature, Vol. 252, No. 5478, Nov 1, 1974, Seiten 83,84
The Journal of Cell Biology, Vol. 59, 1973, Seiten 633-642

Die Akte enthält technische Angaben, die nach dem Eingang der Anmeldung eingereicht wurden und die nicht in dieser Patentschrift enthalten sind.

(73) Patentinhaber: CIBA-GEIGY AG, Postfach,
CH-4002 Basel (CH)

(72) Erfinder: Bürk, Robert R.,, Sichelweg 39,
CH-4103 Bottmingen (CH)
Erfinder: Clopath, Peter, Dr., St. Johanns-Ring 16,
CH-4056 Basel (CH)
Erfinder: Müller, Klaus, Dr., Fürstensteinhof 17,
CH-4107 Ettingen (CH)

(74) Vertreter: Zumstein, Fritz sen., Dr. et al,
Bräuhausstrasse 4, D-8000 München 2 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die vorliegende Erfindung betrifft ein Diagnoseverfahren, insbesondere ein Verfahren zur Diagnose der Atherosklerose.

Seit längerer Zeit wird angenommen, daß die Atherosklerose mit einer Schädigung des arteriellen Endothels ihren Anfang nimmt. Durch Schäden im Endothel wird das subendotheliale Bindegewebe den Einflüssen von Komponenten des zirkulierenden Blutes ausgesetzt. Blutplättchen haften an der freigelegten Oberfläche des Bindegewebes, aggregieren und setzen neben anderen Substanzen einen Wuchsfaktor frei, der, wie nachgewiesen werden konnte, die Proliferation von glatten Muskelzellen in vitro stimuliert. Unter normalen Bedingungen regeneriert sich das Endothel durch eine Kombination von Zellmigration und Zellproliferation und unterbindet innerhalb weniger Tage den Kontakt des Blutes mit dem darunterliegenden Gewebe. Der kumulierende Effekt von kurzen Proliferationsphasen der glatten Muskelzellen ist als mäßige Verdickung der Gefäßintima mit steigendem Alter zu erkennen. Es wird angenommen, daß atherosklerotische Läsionen, d. h. fokale, übermäßige Intima-Verdickungen, sich an Stellen von wiederholten Schädigungen des Endothels entwickeln.

Fortgeschrittene Gefäßläsionen enthalten übermäßig viel Lipide in den innern Teilen, die von fibrösen Teilen mit dichtem Kollagen und hyperplastischen, glatten Muskelzellen bedeckt sind. Gemäß verschiedenen Literaturangaben beschleunigen auch hohe Konzentrationen von Serumlipiden die Bildung von atherosklerotische Läsionen.

Das erfindungsgemäße Diagnoseverfahren beruht nun auf de bisher nicht bekannten Feststellung, daß die Migration von Endothelzellen oder Zellen mit endothelialen Eigenschaften in vitro in Gegenwart von Serum von Versuchstieren, insbesondere Schweinen, die eine atherogene Diät erhielten, wie auch in Gegenwart von Serum von Atherosklerose-Patienten geringer ist als in Gegenwart von Serum normal gefütterter Versuchstiere bzw. von gesunden Personen.

Das erfindungsgemäße Verfahren zur Diagnose der Atherosklerose ist dadurch gekennzeichnet, daß man die Migration von Endothelzellen oder Zellen von endothelartiger Morphologie in an sich bekannter Weise in vitro unter Einhaltung einer Versuchsdauer von weniger als 30 Stunden an entsprechenden Einschicht-Zellkulturen, in denen ein Teil der Zellschicht entfernt wurde, in Gegenwart von Serum der zu untersuchenden Personen mißt und mit der Migration gleicher Zellen in Gegenwart von Standardsera vergleicht, und eine verminderte Migration in Gegenwart von Serum der zu untersuchenden Person als Hinweis auf das mögliche Bestehen von Atherosklerose bei dieser Person erachtet. Im erfindungsgemäßen Verfahren verwendbare Endothelzellen sind beispielsweise solche aus der Schweine-Aorta [vgl. D. De Bono, Nature 252, 83—84 (1974) und J. D. Pearson et al., Biochem. Soc. Transactions 5, 1181—1183 (1977)], und weitere Zellen mit endothelartiger Morphologie sind insbesondere Balb/c 3T3-A31-Zellen, ein Mäuse-Zellstamm, dem endotheliale Eigenschaften zugeschrieben werden [vgl. K. R. Porter G. J. Todaro und V. A. Fonte J. Cell. Biol. 59, 633-642 (1973)]. Wichtig ist jedenfalls, daß die verwendeten endothelartigen Zellen eine Einschicht-Zellkultur bilden.

Die Messung der Zellmigration erfolgt vorzugsweise an Einschicht-Zellkulturen (monolayers), in denen ein Teil der Zellschicht entfernt, d. h. eine oder mehrere sogenannte Wunden angebracht wurden. Um die Beteiligung sowohl der Replikation wie der Migration bei der Wiederherstellung von geschädigtem Endothel durch in vitro Versuche nachzuweisen, massen M. M. Sholley, M. A. Gimbrone und R. S. Cotran, [Laboratory Investigation 36, 18—25 (1977)] den Grad der Wiederbesiedelung von in Einzellenschichten (monolayers) von Endothelzellen menschlicher Nabelschnurvenen angebrachten Kratzern von 0,4-0,5 mm Breite, wobei neben der Zelldichte auch das Ausmaß der DNA-Synthese aufgrund der Aufnahme von $^3$H-Thymidin gemessen wurde und zur Feststellung des Anteils der Migration die Replikation bei einem Teil der Proben durch Bestrahlung ausgeschaltet wurde, was zugleich den Einbau von $^3$H-Thymidin ganz bedeutend herabsetzte. Für die quantitative Erfassung der Migration allein wesentlich geeigneter und einfacher ist indessen eine von R. R. Bürk für eine andere Zielsetzung, die Messung der Stimulierung der Migration von Balb/c 3T3-Zellen durch einen von tumorigenen Zellstamm SV 28 freigesetzten Migrationsfaktor in Serumfreiem Medium, im Vergleich zum Verhalten der normalen Balb/c 3T3-Zellen in Abwesenheit und in Anwesenheit von Serum, in Proc. Nat. Acad. Sci. USA 70, 369—372 (1973) beschriebene Arbeitsweise.

Gemäß dieser Arbeitsweise werden Einschicht-Zellkulturen von Balb/c 3T3 -A31-Zellen in Petrischalen aus Kunststoff in der im nachstehenden Beispiel 1 näher beschriebenen und leicht angepaßten Weise unter Verwendung einer geeigneten Nährlösung, wie Dulbecco's Medium (Gibco H21 HG) enthaltend 10% fötales Kälberserum (Gibco) sowie Penicillin und Streptomycin gezüchtet und anschließend wird mit einer starren Rasierklinge oder Rasierklingenhälfte, je nach Größe der Schale, eine auf einer Seite scharf begrenzte, ca. 1 cm breite, bis in den Kunststoffboden reichende Wunde angebracht. Anschließend wird die Messung der Migration unter Zusatz von frischem Medium mit einem Gehalt von z. B. 4% oder 8% des zu prüfenden Patientenserums bzw. des zum Vergleich dienenden Normalserums durchgeführt. Die Anbringung einer Wunde der angegebenen Größe auf die angegebene Weise ist auch bei der Verwendung anderer Einschicht-Zellkulturen sehr zweckmäßig.

Als Maßstab der Migration dient z. B. die Anzahl der pro mm der scharfen Grenzlinie über diese hinweg in die anfänglich zellfreie Wunde migrierten Zellen. Diese Anzahl wird in den Beispielen als Migrationseinheit bezeichnet. Weil die Zellteilung erst nach 30 Stunden einsetzt, wird die Versuchsdauer auf weniger als 30 Stunden, z. B. auf 22 Stunden beschränkt, womit sich Maßnahmen zur Verhinderung der Replikation erübrigen. Anstelle von Balb/c -3T3-A31-Zellen können beispielsweise auch Endothelzellen aus Schweine-Aorta verwendet werden.

Damit man die Bedeutung der an einer bestimmten Einschicht-Zellkultur festgestellten, verminderten Migrationsaktivität von Patientenserum besser beurteilen kann, ist es zweckmäßig, gleichzeitig an gleich zubereiteten Einschicht-Zellkulturen nicht nur die Migrationsaktivität von Normalserum, sondern auch diejenige von Serum mit bekannter, stark verminderter Migrationsaktivität zu bestimmen, d. h. anstelle von nur einem zwei Standardsera zu verwenden. Solche Standardsera können, anstatt von ausgewählten Personen oder Personengruppen, z. B. auch von speziell, d. h. nicht-atherogen bzw. atherogen, gefütterten Schweinen stammen. Auch in diesem Falle ist aber die Migrationsaktivität der Sera von normalen Personen die maßgebliche Vergleichsgröße, mit der die Schweinesera verglichen und standardisiert und dann als Äquivalent des Humanstandards verwendet werden.

Wie besonders aus dem nachfolgenden Beispiel 4 hervorgeht, eignet sich das erfindungsgemäße Verfahren auch gut zur Kontrolle des Erfolges von therapeutischen und/oder diätetischen Maßnahmen bei bestehender oder vermuteter Atherosklerose.

Die nachfolgenden Beispiele sollen die Erfindung näher erläutern.

### Beispiel 1

Bestimmung der Migrationseinheiten (Zahl der migrierten Zellen pro mm Grenzlinie) der Sera von mit einer atherogenen Diät gefütterten und von normal gefütterten Zwergschweinen.

#### a) Versuchstiere

Als solche dienten insgesamt 25 männliche Zwergschweine. Von diesen erhielten 12 normales Schweinefutter. 11 Schweine erhielten eine atherogene Diät, welche zusätzlich zu normalem Schweinefutter Schmalz, Cholesterin und Erdnußöl enthielt, während 4 Wochen vor der Serumentnahme, und 2 Schweine erhielten dieselbe atherogene Diät während bloß 2 Wochen vor der Serumentnahme.

#### b) Methode

Balb/c 3T3-A31-Zellen, im folgenden als 3T3-B-Zellen bezeichnet, wurden unterhalten durch Weiterzüchten im Verhältnis 1 zu 5 dreimal pro Woche jeweils vor der Konfluenz in Dulbecco's Medium (Gibco H21 HG) mit einem Gehalt an Penicillin und Streptomycin sowie 10% fötalem Kälberserum (Gibco), das durch Erhitzen auf 56°C während 30 Minuten inaktiviert worden war. Je $1 \times 10^5$ 3T3-B-Zellen wurden in 2,5 ml Medium in Kunststoff-Petrischalen (zur Gewebekultur) von 35 mm Durchmesser eingebracht. Nach 5 Tagen Inkubation bei 37°C wurde in jeder Schale eine Wunde angebracht durch leichtes Eindrücken der Hälfte einer starren Rasierklinge (z. B. Personna Gem, reg. Handelsmarke) durch die Zellschicht in den Kunststoffboden zum Markieren einer Grenzlinie, und Bewegen der Klinge mit geringerem Druck senkrecht zu dieser Linie bis auf etwa 10 mm Entfernung davon. Nun wurde das Medium zusammen mit den abgekratzten Zellen abgesaugt. Dann wurden 2,4 ml frisches Medium, bestehend aus Dulbecco's Medium (Gibco H21 HG) mit Penicillin und Streptomycin, gefolgt von 100 µl des zu testenden Serums, entsprechend 4%, zugefügt. Nach 22 Stunden wurde das Medium abgesaugt und die Zellschicht fixiert und zugleich gefärbt mit Hilfe von Leishman's Farbstoff in Methanol (1,5 g Farbstoff pro Liter Methanol, 1,5 ml Lösung pro Schale). Nach 10 Minuten wurden 3,5 ml Wasser zugefügt und nach weiteren 45 Minuten wurde die Schale gründlich mit fließendem Wasser gespült und getrocknet. Zur Auswertung wurden z. B. die Zellen (Kerne) auf 3 Streifen von je 1,2 mm Breite senkrecht zur Grenzlinie gezählt.

#### c) Resultate

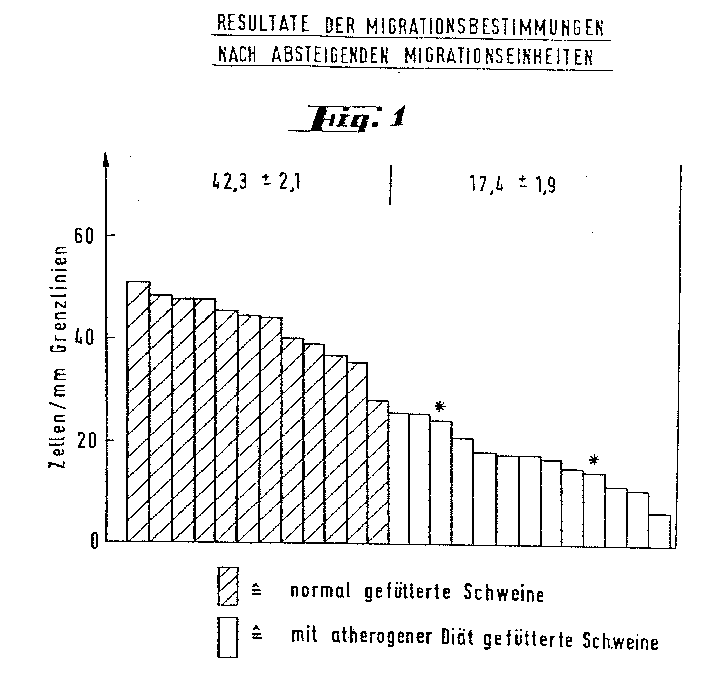

In der Fig. 1 sind die Resultate der Migrationsbestimmungen nach absteigenden Migrationseinheiten (=Anzahl Zellen pro mm Grenzlinie, auf der Ordinatenachse angegeben) zusammengestellt. Schraffierte Balken bedeuten Migrationseinheiten der Sera von normal gefütterten Schweinen und leere Balken die Migrationswerte der Sera von mit atherogener Diät gefütterten Schweinen, wobei die Werte der beiden nur 2 Wochen atherogen gefütterten Schweine mit einem Stern gekennzeichnet sind. Unterteilt man die Sera gemäß ihren Migrationseinheiten in zwei Hälften, so befinden sich die Sera aller normal gefütterten Schweine in der Hälfte mit höheren Migrationseinheiten von durchschnittlich $42,3 \pm 2,1$, und alle Sera der atherogen gefütterten Schweine in der Hälfte mit niederen Migrationseinheiten von durchschnittlich $17,4 \pm 1,9$.

### Beispiel 2

Bestimmung der Migrationseinheiten der Sera von atherosklerotischen und von normalen Personen.

#### a) Herkunft der geprüften Humansera

Es wurden Sera von 29 ambulant behandelten Atherosklerose-Patienten und von 10 normalen Personen verwendet. Bis zur Prüfung wurden die Sera bei −18°C aufbewahrt.

### b) Methode

Gleich wie im Beispiel 1

### c) Resultate

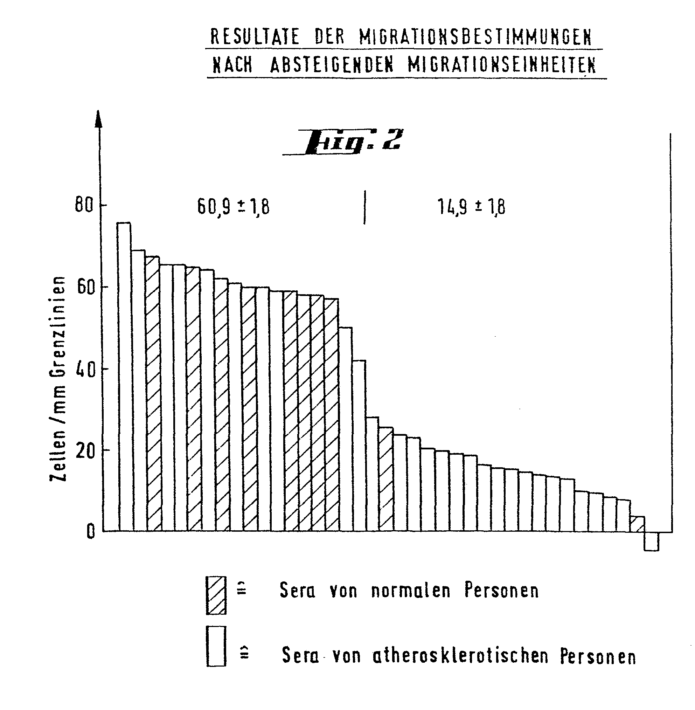

In der Fig. 2 sind die Resultate der Migrationsbestimmungen wiederum nach absteigenden Migrationseinheiten (=Anzahl Zellen pro mm Grenzlinie, auf der Ordinatenachse angegeben) mit schraffierten Balken für Sera von normalen und leeren Balken für Sera von atherosklerotischen Personen zusammengestellt. Bei der Unterteilung der Resultate in Hälften mit hohen bzw. niederen Migrationswerten ergeben sich noch deutlich stärker verschiedene Durchschnittswerte von 60,9±1,8 bzw. 14,9±1,8 Migrationseinheiten. Die Sera von zwei normalen Personen zeigen Migrationswerte in der unteren Hälfte, andererseits liegen die Migrationswerte der Sera von 8 Atherosklerose-Patienten nicht in der unteren Hälfte oder im unteren Teil der oberen Hälfte. Das Gesamtbild ist also nicht ganz so einheitlich wie im Beispiel 1, indessen wurden bei den Sera von insgesamt 19 der 29 Patienten eindeutig niedrigere Migrationseinheiten festgestellt als bei der Gesamtheit der normalen Personen; stark erniedrigte Migrationseinheiten sind somit ein charakteristisches Merkmal von ca. $^2/_3$ der Patienten, während nur bei 2, d. h. $^1/_5$ der normalen Personen ungefähr gleich niedrige Migrationseinheiten festgestellt wurden. Bemerkenswert ist auch die deutliche Trennung von erniedrigten und »normalen« Migrationseinheiten, da nur 2 von insgesamt 29 Patientensera Werte ergaben, die nicht den »normalen« oder erniedrigten zugeordnet werden können.

### Beispiel 3

Vergleich der Migrationsaktivität von Humansera auf 3T3-B-Zellen (vgl. Beispiel 1) und auf Endothelzellen von Schweine-Aorta.

### a) Herkunft der Sera

Als Humansera wurden zwei mit hoher Migrationsaktivität und drei mit niedriger Migrationsaktivität in den Versuchen gemäß Beispiel 2 verwendet.

### b) Verwendete Zellkulturen

Reine Kulturen von Endothelzellen von Schweine-Aorta wurden wie folgt bereitet: Von den Aorten von frischgeschlachteten, 12 Wochen alten Schweinen wurden die Endothelzellen mit Hilfe von Kollagenase und mit den geringstmöglichen mechanischen Eingriffen entfernt [vgl. J. D. Pearson et al., Biochem. Soc.

Transactions 5, 1181−1183 (1977)] und zusammen mit Waymouth's Medium 752/1, enthaltend 20% fötales Kälberserum, in Petrischalen gebracht. Nach der Konfluenz wurden je $2 \times 10^5$ Zellen in Petrischalen aus Kunststoff von 35 mm Durchmesser mit 2,5 ml Waymouth's Medium, ergänzt durch 10% fötales Kälberserum, weiterkultiviert. Nach 5 Tagen wurden die Zellkulturen zur Bestimmung der Migrationsaktivität verwendet, vgl. c).

Als 3T3-B-Zellkulturen wurden die bereits im Beispiel 1 beschriebenen verwendet.

### c) Methode

Bei den 3T3-B-Zellkulturen war diese gleich wie im Beispiel 1 beschrieben. Bei den Endothelzellkulturen aus Schweine-Aorta wurde in folgenden Punkten von der Methode des Beispiels 1 abgewichen: Als Medium wurde Waymouth's 752/1 verwendet. Die Zelldichte war während des Versuchs ungefähr doppelt so groß in den Schalen mit 3T3-B-Zellen, d. h. ca. $10_5$ pro cm² statt ca. $5 \times 10^4$ pro cm²; die in der Tabelle angegebenen Migrationseinheiten beziehen sich somit ebenfalls auf eine ungefähr doppelt so große Zellenzahl an der Grenzlinie.

### d) Resultate

Wie aus der nachfolgenden Tabelle hervorgeht, verhielten sich die geprüften Humansera bezüglich ihrer Migrationsaktivität gegenüber den Endothelzellen aus Schweine-Aorta im Prinzip gleich wie gegenüber 3T3-B-Zellen. Die Zahl der migrierten Endothelzellen aus Schweine-Aorta war in allen Fällen niedriger als bei 3T3-B-Zellen, indessen waren die Unterschiede in den Migrationsaktivitäten der verschiedenen Sera noch ausgeprägter, mit einem durchschnittlichen Verhältnis der Migrationseinheiten von niedrigaktiven zu normalaktiven Humansera von 2,9 zu 19,8 bzw. 1 zu 6,8, gegenüber 14,8 zu 58,2 bzw. 1 zu 3,9 bei 3T3-B-Zellen. Daraus kann gefolgert werden, daß sich Endothelzellen aus Schweine-Aorta für die Verwendung im erfindungsgemäßen Diagnoseverfahren ebenso eignen wie die in den Beispielen 1 und 2 ausschließlich verwendeten 3T3-B-Zellen.

Tabelle zu Beispiel 3

| Serum No | Migrationseinheiten, bestimmt an: | |
| --- | --- | --- |
| | Endothelzellen von Schweine-Aorta | 3T3-B-Zellen |
| 9 | 1,6 | 14,9 |
| 12 | 3,2 | 15,2 |

Tabelle zu Beispiel 3 (Fortsetzung)

| Serum No | Migrationseinheiten, bestimmt an: | |
| | Endothelzellen von Schweine-Aorta | 3T3-B-Zellen |
| --- | --- | --- |
| 13 | 3,8 | 14,3 |
| 29 | 18,0 | 57,6 |
| 34 | 21,6 | 58,7 |

## Beispiel 4

Zeitlicher Verlauf der Migrationsaktivität der Sera von Schweinen mit wechselnder Diät.

### a) Versuchstiere

Als solche wurden zwei weibliche Zwergschweine verwendet, welche bei Versuchsbeginn 4 Monate alt waren und je 12 kg wogen.

### b) Fütterungsweise

Die Tiere erhielten zunächst während 4 Wochen normales Schweinefutter, dann während 4 Wochen atherogenes Futter, d. h. Schweinefutter mit Zusatz von Schmalz, Cholesterin und Erdnußöl, und schließlich während 8 Wochen wiederum normales Schweinefutter.

### c) Bestimmung der Migrationsaktivität

Einmal wöchentlich wurde Serum gewonnen bis zu Auswertung bei −18°C aufbewahrt. Die Migrationsaktivität der Sera wurde als Anzahl Migrationseinheiten völlig analog Beispiel 1 gemessen. Zusätzlich wurde jeweils auch der Cholesterinspiegel auf übliche Weise bestimmt.

### Resultate

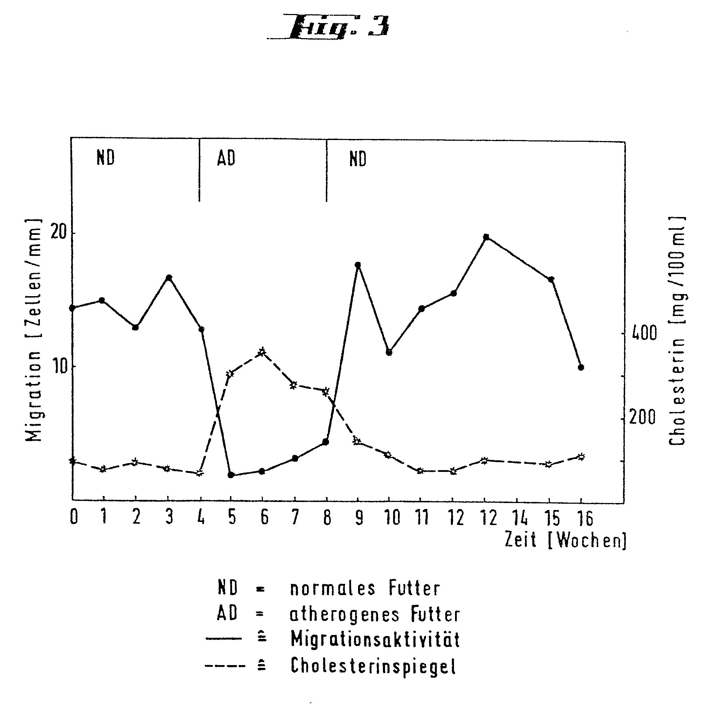

Die wöchentlich (Anzahl Wochen auf der Abszissenachse angegeben) festgestellten Migrationseinheiten als Durchschnittswerte der Sera beider Schweine (Anzahl Zellen pro mm Grenzlinie, auf der linken Ordinatenachse angegeben) und die zugehörigen Cholesterinspiegel (in mg/100 ml, auf der rechten Ordinatenachse angegeben) sind in der Fig. 3 zusammengestellt. Die entsprechenden Kurven zeigen eindrücklich, daß nach dem Übergang von normalen (ND) zu atherogenem Futter (AD) die Migrationsaktivität (ausgezogene Linien) mindestens so rasch absinkt wie der Cholesterinspiegel (in mg/100 ml, gestrichelte Linien) ansteigt, und nach dem Rückwechsel zur normalen Fütterung wieder, wenn auch etwas weniger rasch, die ursprünglichen Werte erreicht werden.

Eine zweckmäßige Ausrüstung zur Durchführung des erfindungsgemäßen Diagnoseverfahrens, welche die hierzu benötigten, in der Beschreibung und den Beispielen erwähnten Materialien und Geräte enthält, hat

— geeignete Zellen für die Einschicht-Zellkultur, z. B. ausgewählt vom Stamm Balb/c 3T3-A31, oder ausgewählte Endothelzellen von Arterien von Schweinen oder Rindern, oder ausgewählte Endothelzellen von menschlicher Nabelvene;
— tierisches Serum, geeignet zur Vorbereitung der Einschicht-Zellkultur, z. B. ein ausgewähltes, geprüftes foetales Kalbsserum oder Pferdeserum;
— geeignetes Standardserum mit normaler Migrationsaktivität, z. B. Serum von speziell gefütterten Schweinen oder ein ausgewähltes menschliches Serum;
— geeignetes Standardserum mit erniedrigter Migrationsaktivität, z. B. Serum von speziell gefütterten Schweinen oder ein ausgewähltes menschliches Serum;
— geeignetes Medium zur Kultivierung der Zellen, wie z. B. ein modifiziertes Medium nach Eagle, oder ein Medium nach Dulbecco, oder Waymouth's Medium; sowie gegebenenfalls
— geeignete, sterile Kulturschalen aus Plastikmaterial;
— Klingen zur Erzeugung der Wunde, besonders geeignet bezüglich Eigenschaften und Größe, z. B. entsprechend einer halbierten oder ganzen Rasierklinge;
— Vorrichtung, geeignet zur Halterung der Klinge für das Setzen der Wunde;
— Vorrichtung, geeignet zur Halterung der Kulturschalen beim Setzen der Wunde;
— ein geeignetes Mittel zum Anfärben der Zellkerne, wie z. B. eine 0,15prozentige Lösung von Leishman's Farbstoff in Methanol;
— eine ausführliche Beschreibung für die Durchführung und Auswertung des Diagnoseverfahrens.

## Patentansprüche

1. Verfahren zur Diagnose der Atherosklerose, dadurch gekennzeichnet, daß man die Migration von Endothelzellen oder Zellen von endothelartiger Morphologie in an sich bekannter Weise in vitro unter Einhaltung einer Versuchsdauer von weniger als 30 Stunden an entsprechenden Einschicht-Zellkulturen, in denen ein Teil der Zellschicht entfernt wurde, in Gegenwart von Serum der zu untersuchenden Person mißt und mit der Migration gleicher Zellen in Gegenwart von Standardsera vergleicht, und eine verminderte Migration in Gegenwart von Serum der zu untersuchenden Person als Hinweis auf das mögliche Bestehen von Atherosklerose bei dieser Person erachtet.

2. Verfahren nach Anspruch 1, dadurch gekennzeichnet, daß man die Zellmigration an Einschicht-Zellkulturen von Endothelzellen aus Schweine-Aorta mißt.

3. Verfahren nach Anspruch 1, dadurch gekennzeichnet, daß man die Zellmigration an Einschicht-Zellkulturen aus Balb/c 3T3-A31-Zellen als Zellen mit endothelartiger Morphologie mißt.

4. Verfahren nach Anspruch 2, dadurch gekennzeichnet, daß man als Medium für die Einschicht-Zellkulturen Waymouth's Medium 752/1 verwendet.

5. Verfahren nach Anspruch 3, dadurch gekennzeichnet, daß man als Medium für die Einschicht-Zellkulturen Dulbecco's Medium verwendet.

6. Verfahren nach Ansprüchen 5 und 6, dadurch gekennzeichnet, daß man das zu prüfende Patientenserum bzw. das zum Vergleich dienende Normalserum dem Medium in einer Konzentration von 4% bis 8% beifügt.

## Claims

1. A method of diagnosing atherosclerosis, which method comprises determining in a manner known per se in vitro the migration of endothelial cells or of cells of endothelial morphology, maintaining a test duration of less than 30 hours, on corresponding single-layer cell cultures in which a part of the cell layer has been removed, in the presence of serum of the test person, and comparing said migration with the migration of identical cells, in the presence of standard sera, and taking a reduction in migration, in the presence of serum of the test person, as indication of the possible occurrence of atherosclerosis in said person.

2. A method according to claim 1, which comprises determining the cell migration on single-layer cell cultures of endothelial cells of pig aorta.

3. A method according to claim 1, which comprises determining the cell migration on single-layer cell cultures of Balb/c 3T3-A31 cells as cells having endothelial morphology.

4. A method according to claim 2, which comprises using Waymouth's medium 752/1 as medium for the singlelayer cell cultures.

5. A method according to claim 3, which comprises using Dulbecco's medium as medium for the single-layer cell cultures.

6. A method according to either of claims 5 or 6, which comprises adding the test serum or the normal serum used for comparison purposes to the medium in a concentration of 4% to 8%.

## Revendications

1. Procédé pour le diagnostic de l'athérosclérose, caractérisé en ce que l'on mesure la migration de cellules endothéliales ou de cellules de morphologie de type endothélial, de manière connue en soi, in vitro, en respectant une durée d'essai inférieure à 30 heures, sur des cultures de cellules correspondantes à une couche dans lesquelles une partie de la couche de cellules a été éliminée, en présence de sérum du patient et on compare avec la migration de cellules identiques en présence de sérums normaux, et on considère une migration amoindrie en présence de sérum du patient comme une indication de l'existance possible d'athérosclérose chez ce patient.

2. Procédé selon la revendication 1, caractérisé en ce que l'on mesure la migration cellulaire sur des cultures de cellules à une couche de cellules endothéliales d'aorte de porc.

3. Procédé selon la revendication 1, caractérisé en ce que l'on mesure la migration cellulaire sur des cultures de cellules à une couche de cellules Balb/c 3T3-A31 en tant que cellules à morphologie de type endothélial.

4. Procédé selon la revendication 2, caractérisé en ce que l'on utilise en tant que milieu pour la culture de cellules à une couche du milieu Waymouth 752/1.

5. Procédé selon la revendication 3, caractérisé en ce que l'on utilise en tant que milieu pour la culture de cellules à une couche du milieu Dulbecco.

6. Procédé selon les revendications 5 et 6, caractérisé en ce que l'on ajoute le sérum du patient et le sérum normal serevant de témoin au milieu à une concentration de 4 à 8%.

RESULTATE DER MIGRATIONSBESTIMMUNGEN
NACH ABSTEIGENDEN MIGRATIONSEINHEITEN

## Fig. 1

$42,3 \pm 2,1$      $17,4 \pm 1,9$

◩ ≙ normal gefütterte Schweine

☐ ≙ mit atherogener Diät gefütterte Schweine

## RESULTATE DER MIGRATIONSBESTIMMUNGEN
## NACH ABSTEIGENDEN MIGRATIONSEINHEITEN

Fig. 2

60,9 ± 1,8    |    14,9 ± 1,8

Zellen / mm Grenzlinien

▨ ≙  Sera von normalen Personen

☐ ≙  Sera von atherosklerotischen Personen

# *Fig. 3*

ND = normales Futter
AD = atherogenes Futter
——— ≙ Migrationsaktivität
---- ≙ Cholesterinspiegel